# FASCICULE DE BREVET EUROPEEN

(11) **EP 2 020 896 B1**
(45) Date de publication et mention de la délivrance du brevet: **27.03.2013**
(21) Numéro de dépôt: 07731377.3
(22) Date de dépôt: 27.04.2007
(51) Int. Cl.: A61B 1/00, G02B 21/00, G02B 23/24

(54) **TETE OPTIQUE MINIATURISEE A HAUTE RESOLUTION SPATIALE ET HAUTE SENSIBILITE, NOTAMMENT POUR L'IMAGERIE DE FLUORESCENCE CONFOCALE FIBREE**
MINIATURISIERTER OPTISCHER KOPF MIT HOHER RÄUMLICHER AUFLÖSUNG UND HOHER SENSITIVITÄT, INSBESONDERE FÜR KONFOKALE FASER-FLUORESZENZ-DARSTELLUNG
MINIATURIZED OPTICAL HEAD WITH HIGH SPATIAL RESOLUTION AND HIGH SENSITIVITY, ESPECIALLY FOR FIBRED CONFOCAL FLUORESCENCE IMAGING

(30) Priorité: 05.05.2006 FR 0604019
(43) Date de publication de la demande: 11.02.2009
(73) Titulaire: Mauna Kea Technologies, 75010 Paris (FR)
(72) Inventeur: GENET, Magalie, F-78280 Guyancourt (FR); MATHIEU, Gilles, 34400 Lunel (FR); VIELLEROBE, Bertrand, F-94130 Nogent sur Marne (FR); DOUSSOUX, François, F-75009 Paris (FR); BOULAROT, Nicolas, F-94500 Champigny sur Marne (FR); LAVILLONNIERE, Nicolas, F-94100 Saint Maur des Fosses (FR)
(74) Mandataire: Pontet, Bernard
(86) Numéro de dépôt international: PCT/FR2007/000723
(87) Numéro de publication internationale: WO 2007/128909

(56) Documents cités:
- WO-A-03/056379
- WO-A2-2004/065994
- US-A- 3 700 311
- US-A- 5 469 299
- US-A1- 2004 252 380

## Description

La présente invention concerne une tête optique miniaturisée prévue pour équiper une extrémité distale d'un faisceau de fibres optiques souples, ladite tête étant destinée à être placée au contact d'une surface d'analyse et adaptée à focaliser un signal d'excitation véhiculé par ledit faisceau de fibres en un point focal d'excitation pouvant être situé à différentes profondeurs par rapport à la surface de contact de la tête. La tête optique est également adaptée à prélever un signal rétroémis provenant du point focal d'excitation subsurfacique pour qu'il soit acheminé par le faisceau de fibres notamment vers des moyens de détection et des moyens d'analyse et de traitement numérique de signal.

Les domaines d'application concernés sont les dispositifs d'analyse subsurfacique à caractère confocal, les signaux véhiculés pouvant être notamment du domaine de l'imagerie et/ou de la spectroscopie suivant la ou les sources d'excitation et les moyens de détection mis en oeuvre. Le caractère confocal résulte de l'utilisation de la même fibre pour véhiculer le signal d'excitation et le signal rétroémis, et d'une dimension spatiale très réduite de la fibre. Il peut s'agir d'analyses biologiques in situ, sur l'homme ou l'animal, externes par exemple du domaine de la dermatologie, ou internes et accessibles à l'aide d'un canal opérateur d'endoscope dans lequel on peut introduire le faisceau de fibres optiques et la tête optique. Il peut s'agir également d'analyses cellulaires réalisées ex vivo sur des prélèvements. En outre encore, la tête optique peut être mise en oeuvre pour l'analyse de l'intérieur d'un dispositif manufacturé.

Actuellement sont visés les domaines médicaux de la gastro-entérologie, la pneumologie, la gynécologie, l'urologie, l'ORL, la dermatologie, l'ophtalmologie, la cardiologie et de la neurologie.

Les moyens d'analyse et de traitement de signal prévus du côté de l'extrémité proximale du faisceau de fibres optiques permettent de restituer une image ou un graphe interprétable par un utilisateur.

Le document WO 2004/065994 décrit une tête optique miniaturisée pour un microscope confocal.

Le document US 2004 / 02 522 380 décrit une lentille pour objectif ayant une transmittance d'au moins 50% à une longueurs d'onde de 300 nm pour une épaisseur de 10 mm.

On connaît le document WO 03/056379 décrivant une tête optique miniaturisée prévue pour équiper l'extrémité distale d'un guide d'image. Cette tête optique consiste en un tube porte-optique de section circulaire au sein duquel sont introduits d'un côté la partie terminale distale du guide d'image et de l'autre des moyens optiques de focalisation. Les moyens optiques comprennent des lentilles disposées dans des plans extra-focaux. Les lentilles sont disposées de façon à optimiser le rapport signal à bruit en minimisant la réflexion parasite en sortie de guide d'image, en optimisant le taux de couplage en retour et en optimisant la transmission de l'ensemble de la tête. La présente invention a pour but une tête optique offrant une bonne qualité d'image pour des systèmes de fluorescence notamment.

On connaît également le document de A.R. Rouse, A. Kano, J.A. Udovich, S.M. Kroto and A.F. Gmitro, "Design and demonstration of a miniature catheter for a confocal microendoscope", Applied Optics, vol. 43, n° 31, pp. 5763-5771, 2004. Ce document décrit une tête optique qui se place à la partie distale d'un guide d'image. Cette tête optique présente les particularités suivantes :
- Grandissement= 1.6, ce qui fait assez peu et ne devrait pas permettre d'obtenir une grande résolution latérale;
- Ouverture numérique sur le tissu = 0.46, ce qui est un inconvénient pour la collection des photons provenant de la fuorescence du tissu; La sensibilité ne peut pas être très importante avec une telle solution optique;
- Diamètre de la tête = 3mm;
- Longueur de la tête = 13mm, ce qui est compatible avec le passage par le canal opérateur d'un endoscope;
- Résolution axiale ponctuelle = 10 µm, ce qui est une valeur assez grande pour une tête optique comportant un design optique relativement compliqué;
- Résolution axiale planaire = 25 µm (c'est à dire pour un champ imagé entier), ce qui est une valeur importante pour une tête optique comportant un design optique compliqué;
- Résolution latérale = 3 µm, assez dégradée pour une tête optique de ce type.

La tête optique selon l'art antérieur n'a pas de performances optiques permettant de la qualifier de tête optique de haute résolution et de haute sensibilité. Par ailleurs, le document Rouse décrit un système de balayage ligne par ligne directement et non un système de balayage point par point en injectant le faisceau optique tour à tour dans chacun des fibres optiques constituant le guide d'image.

La présente invention a pour but de remédier aux inconvénients de la tête optique de l'art antérieur en proposant une tête optique miniature de grande qualité optique : haute résolution (résolution latérale et résolution axiale) et haute sensibilité, notamment pour l'imagerie confocale de fluorescence fibrée à balayage laser in vivo. La miniaturisation doit permettre notamment l'insertion de l'ensemble guide d'image (faisceau de fibres optiques ou "bundle") et tête optique dans le canal opérateur d'un endoscope ou bien d'être le moins invasif possible sur le petit animal. La nécessité d'une grande qualité optique s'inscrit dans la volonté de détecter avec une très bonne résolution des objets de petites tailles (membranes, dendrites, organelles...) et avec une très bonne sensibilité. Ainsi, il serait possible de détecter de manière plus aisée des objets situés plus en profondeur dans le tissu.

Un autre but de l'invention est une tête de grande qualité particulièrement adaptée pour des acquisitions en temps réel.

On atteint au moins l'un des objectifs précités avec une tête optique miniaturisée selon la revendication 1.

Plus précisément, le bloc de correction des aberrations sphériques corrig également les aberrations de coma et d'astigmatisme.

Avantageusement, le faisceau de fibres optiques est balayé par le faisceau laser en temps réel de façon à acquérir au moins douze images par seconde.

Avec la tête optique selon l'invention, on obtient des performances de qualité de front d'onde et d'imagerie à grande ouverture numérique, de préférence supérieure à 0.8, sans nécessairement ajout de ménisques correcteurs : une seule lentille divergente ainsi disposée permet d'atteindre de très bonnes performances. D'une façon générale, on peut considérer qu'une ouverture numérique est grande à partir d'une valeur de 0.6. Une grande ouverture numérique image permet d'obtenir une haute sensibilité. Elle permet dans un premier temps de focaliser le faisceau d'excitation provenant de la fibre d'illumination dans un volume d'excitation très confiné, ce qui permet de maximiser la densité d'énergie au point focal et ainsi d'exciter l'échantillon ou tissu de manière optimale. Par exemple, elle permet également de maximiser la collection du nombre de photons de fluorescence qui sont émis de manière isotrope dans l'échantillon. Une ouverture numérique supérieure à 0.8 peut être obtenue par l'utilisation d'une lentille ou d'un groupe de lentilles convergentes présentant de très petits rayons de courbure comme par exemple un groupe de lentilles fortement convergentes se terminant par une lentille boule ou demi-boule. La lentille demi-boule présente l'avantage d'être fabricable avec de très faibles diamètres, et d'assurer un bon contact avec l'échantillon afin de minimiser le mouvement inhérent à l'opérateur ou au sujet analysé et de réaliser une bonne adaptation d'indice de manière à s'affranchir du signal provenant de la surface d'analyse.

Par ailleurs, la tête optique selon l'invention présente un fort grandissement. Le grandissement de la tête optique se calcule comme le rapport entre :
- l'ouverture numérique du premier bloc optique servant à la correction des aberrations dont l'une des fonctions est d'adapter l'ouverture numérique des fibres optiques; sa valeur est comprise entre 0.3 et 0.32; et
- l'ouverture numérique (>0.8) du second bloc optique de focalisation, côté tissu, celui dont la fonction principale est la focalisation du faisceau dans le tissu.

Ainsi, les valeurs de grandissement dans le cas présent sont comprises entre 2.5 et 4. Cela permet d'obtenir une résolution spatiale bien meilleure que les autres têtes optiques utilisées dans l'art antérieur. Cet avantage important sur la résolution est effectif dans la mesure où la tête optique n'est pas aberrante optiquement.

Plus précisément, le bloc de correction des aberrations sphériques comprend une troisième lentille convergente; les épaisseurs des première et troisième lentilles convergentes étant déterminées de façon à corriger l'aberration sphérique cumulée sur les dioptres convergents desdites lentilles convergentes de la tête optique.

Avantageusement, l'épaisseur de la troisième lentille convergente est déterminée en outre de façon à imager la pupille sur la seconde lentille convergente, ceci pour minimiser les aberrations.

Selon un mode de réalisation de l'invention, l'épaisseur, le rayon de courbure (ou puissance optique) et la nature de cette troisième lentille (L2) sont adaptées de façon à imager la pupille dans un plan situé le plus proche possible dudit faisceau de fibres optiques. Imager la pupille le plus proche possible de la sortie des fibres permet d'avoir une image de petite taille sur le plan pupillaire, donc un diamètre de la tête optique réduit, ce qui va dans le sens d'une miniaturisation optimisée.

De préférence, le verre de la troisième lentille est déterminé avec une constringence suffisamment faible pour minimiser la puissance de correction chromatique nécessaire, et avec un indice de réfraction suffisamment élevé pour limiter le terme d'aberration sphérique et de coma. On peut par exemple utiliser des verres de type "FLINT".

Selon une caractéristique avantageuse de l'invention, ledit doublet est placé dans un plan pupillaire donnant des caractéristiques de faibles aberrations, notamment de faible astigmatisme. Avantageusement, on détermine une épaisseur suffisante de la seconde lentille convergente pour éloigner la lentille divergente à la juste distance de la pupille pour laquelle les conditions d'achromatisation latérales coïncident avec les conditions d'achromatisation axiale. En outre, la cambrure ou courbure de la lentille divergente est centrée sur la pupille de façon à minimiser l'astigmatisme en sortie de la lentille divergente; la pupille étant une surface (pas nécessairement plane) commune aux faisceaux issus de tous les points du champ. De manière générale, on raisonne toujours dans le sens de propagation de la lumière : à l'excitation, la lumière est issue des fibres. Le champ est donc le faisceau lui même. Les points du champ sont alors les fibres elles mêmes. Si on raisonne à la collection, on revient de l'échantillon, le champ est le champ observé.

Par ailleurs, le doublet comporte un couple de verres de faible écart d'indice mais avec un fort écart de dispersion chromatique de façon à compenser les aberrations chromatiques des lentilles convergentes. On peut porter le choix des verres dans le doublet sur un couple de verres de faible écart d'indice pour minimiser les termes d'aberration géométrique induit dans l'interface entre les deux lentilles du doublet, mais avec un fort écart de dispersion chromatique. Le doublet ainsi obtenu est surcorrigé du chromatisme axial pour compenser les aberrations chromatiques des éléments convergents. A titre d'exemple, on peut utiliser le couple de verres suivants : verre1 (LAK21, nd = 1,6405, Vd = 60,1) et verre2 (SF6, nd = 1,8052, Vd = 25,4), qui présentent un faible écart d'indice, et un fort écart de constringence.

Les première et troisième lentilles convergentes peuvent s'obtenir chacun à partir d'une lentille ayant un petit rayon et un grand rayon, ledit grand rayon étant rendu plan de façon à obtenir une lentille plan-convexe.

Selon l'invention, la première lentille convergente est conçue et disposée de façon à rejeter l'image de la pupille suffisamment loin en avant pour minimiser l'astigmatisme généré par la lentille boule ou demi-boule dans le champ imagé.

La qualité optique de la tête selon l'invention est un paramètre permettant d'obtenir une image à haute résolution spatiale. Cette qualité image est très proche de la limite de diffraction. Le WFE ("Wave Front Error" en langue anglaise) est de l'ordre de λ/15 au centre de champ et de λ/10 en bord de champ. Ces valeurs de WFE permettent de s'affranchir de la présence d'aberrations optiques trop importantes qui pourraient dégrader :
1. la résolution latérale qui est évaluée par l'énergie encerclée. Par définition, pour résoudre une tâche de diamètre φ, il faut que 50% de l'énergie soit au minimum contenue dans ce diamètre. En l'occurrence pour une sonde hautement résolue, 50% de l'énergie doit être contenue dans un diamètre de l'ordre du micron. Dans ce cas, ce n'est pas la tête optique qui limite la résolution latérale, mais la distance entre les coeurs des fibres constituant le guide d'image. La résolution latérale est donnée par la distance inter-coeurs divisée par le grandissement de la tête optique, soit une résolution optique latérale inférieure à 1,5 µm pour des sondes utilisées selon la présente invention, ce qui est bien meilleur que les systèmes de l'art antérieur.
2. la résolution axiale qui est fonction de l'ouverture numérique image, et qui peut être dégradée par l'apparition d'aberration sphérique. Afin d'assurer par exemple une image confocale de fluorescence hautement résolue, la résolution axiale est de préférence inférieure à 5 µm. Etant donné que l'obtention d'une haute sensibilité passe par l'utilisation d'une grande ouverture numérique et donc de lentilles convergentes de faibles rayons de courbure décrites avec des angles de champ importants qui induisent des aberrations, l'obtention d'une très bonne résolution qu'elle soit latérale ou axiale passe par l'utilisation d'un bloc optique amont (situé entre le guide d'image et le bloc optique de focalisation) constitué de lentilles correctrices permettant de corriger les aberrations telles que la coma et l'astigmatisme qui dégradent la résolution latérale, et l'aberration sphérique qui dégrade la résolution axiale.

Selon l'invention, la lentille demi-boule peut être fabriquée dans une bille, dont la face de sortie plane est obtenue par usure sur un polissoir plan.

L'épaisseur de la lentille demi-boule peut être ajustée pour obtenir une frontale prédéterminée de la tête optique. En outre, la position axiale de la lentille demi boule, c'est à dire l'épaisseur d'air entre la lentille qui la précède (première lentille convergente) et la lentille demi boule, est déterminée en fonction de l'épaisseur de la dite lentille demi-boule (L5) afin d'optimiser les performances optiques (résolutions axiale et latérale, ...) de la tête optique pour ladite frontale.

Avantageusement, la tête optique selon l'invention comprend en outre une lame à faces planes pour rejeter la réflexion parasite s'opérant en sortie du faisceau de fibres optiques.

Selon l'invention, le signal collecté par la tête optique en provenance de l'échantillon peut être un signal de fluorescence ou un signal de réflectance. Pour l'obtention d'image en mode de fluorescence, la tête optique est achromatique dans une bande spectrale entre 450nm et 800nm. Une telle achromaticité sur une large bande spectrale permet une utilisation également en spectroscopie et en multimarquage. Cette tête optique est donc également compatible avec une utilisation en imagerie de réflectance puisque celle-ci s'opère dans le rouge proche infra rouge couvert par la large bande spectrale de travail de la tête optique.

En ce qui concerne l'échantillonnage du tissu, la tête optique selon l'invention permet d'obtenir une bonne fonction d'étalement de point (PSF) ou tache focale, de l'ordre du micron. De plus, le grandissement optique est tel que la résolution image est meilleure que pour les sondes classiques. Un tel échantillonnage selon l'invention permet d'obtenir des images de meilleure résolution.

La tête optique selon l'invention permet notamment d'atteindre les performances suivantes :
- un balayage laser avec acquisition rapide en temps réel, de quelques images par secondes à au moins 12 images par seconde;
- une miniaturisation suffisante : diamètre compris entre 2 et 4,5mm et une longueur de tête comprise entre 10 et 27mm;
- une tête confocale avec une très bonne résolution axiale (<5 µm) et latérale(<1.5 µm);
- une grande sensibilité pour collecter des photons de fluorescence avec une ouverture numérique côté tissu > 0.8.

La présente invention est notamment remarquable par le fait qu'elle permet de concilier des critères difficilement compatibles, à savoir une acquisition par balayage laser en temps réel avec une grande sensibilité. De même, une miniaturisation suffisante avec une grande résolution (grande ouverture numérique).

D'autres avantages et caractéristiques de l'invention apparaîtront à l'examen de la description détaillée d'un mode de mise en oeuvre nullement limitatif, et des dessins annexés, sur lesquels :
- La figure 1 est un schéma simplifié des éléments optiques d'une tête miniature avec un grandissement de 2.5 selon l'invention,
- La figure 2 est un schéma simplifié des éléments optomécaniques de la tête optique de la figure 1,
- La figure 3 est un schéma simplifié des éléments optiques d'une tête miniature avec un grandissement de 4.

On va maintenant décrire une tête opto-mécanique miniaturisée prévue pour équiper l'extrémité distale d'un faisceau de fibres optiques souples, destinée à être mise au contact de la surface d'analyse et comprenant des moyens optiques permettant de focaliser le faisceau d'excitation à une profondeur donnée sous la surface d'analyse et de collecter de manière optimale le signal de fluorescence provenant du même volume que le volume d'excitation (Caractère confocal).

D'une façon générale, la tête opto-mécanique est constituée :
- d'une association de plusieurs optiques (réfractives, diffractives ou à gradient d'indice) constituant un ensemble achromatique et permettant d'illuminer la surface d'analyse tout en assurant une bonne qualité optique nécessaire à l'obtention d'une image de fluorescence confocale hautement résolue, et
- d'un ensemble mécanique permettant :
   1. de maintenir et d'aligner les optiques avec des tolérances de centrage et de tilt très serrées afin de ne pas s'écarter de la position nominale, et ainsi ne pas induire d'aberrations pouvant dégrader la qualité optique et par conséquent la résolution spatiale, et
   2. de connecter la tête opto-mécanique avec le guide d'image.

Cet ensemble mécanique peut être réalisé dans un matériau métallique biocompatible comme par exemple l'inox 316L et avec des épaisseurs minimales afin de ne pas obturer les faisceaux décrits par les fibres en bord du guide d'image, et ne pas augmenter de manière trop importante les dimensions de la tête optique. En raison de l'encombrement réduit de la tête optique selon l'invention, les pièces mécaniques maintenant les optiques présentent une épaisseur de "peau" (Différence entre diamètre intérieur et diamètre extérieur) minimale (inférieure à 300 µm). Par ailleurs, afin de ne pas dégrader la qualité optique ayant un impact direct sur la sensibilité et la résolution spatiale, l'assemblage de la tête est réalisé avec des tolérances de centrage et de tilt de quelques microns. Ceci nécessite par exemple un réglage des lentilles sur banc optique.

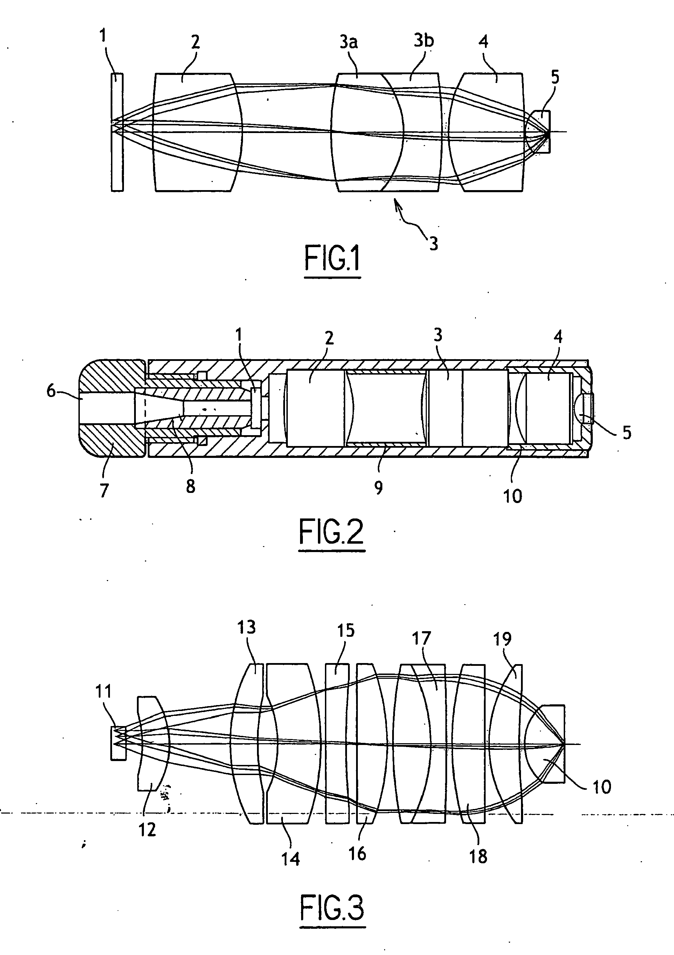

De manière plus précise, sur la figure 1 on voit un schéma simplifié d'un ensemble d'éléments optiques d'une tète optique selon l'invention. Ce dispositif permet d'élaborer une tête miniature comme représenté sur la figure 2 avec un grandissement de 2.5.

Sur la figure 1, l'ensemble optique est constitué d'un bloc de focalisation constitué d'un doublet 3, et une lentille convergente 4 une lentille convergente demi-boule 5. Il comprend également une lentille correctrice en amont représentée par la lentille convergente 2. Le bloc optique de focalisation a pour fonction de focaliser le faisceau d'excitation en un point focal d'excitation situé dans un plan d'analyse subsurfacique. A titre d'exemple, ce bloc optique de grandissement égal à 2.5, soit une ouverture numérique côté tissu de 0.8, est constitué du doublet 3 achromatique en verre N-LAK21 et N-SF6, de la lentille 4 bi-convexe en verre SK16, et de la lentille demi-boule 5 en verre BK7 permettant d'assurer une focalisation importante et un bon contact avec la surface d'analyse.

Dans cet exemple, le bloc optique de correction permettant de corriger les aberrations induites par le bloc optique de focalisation est constitué de la lentille 2 bi-convexe en verre SK16. Ce bloc optique de correction a au moins trois fonctions. La première fonction est de créer une aberration sphérique apte à compenser les aberrations sphériques introduites par d'autres lentilles placées en aval dans le sens de l'excitation; ceci est notamment obtenu grâce une épaisseur de verre importante de la lentille L2; l'avantage en est qu'il n'est pas nécessaire d'ajouter une nouvelle lentille pour corriger les aberrations des lentilles placées en aval. La seconde fonction est que le verre de la troisième lentille L2 est déterminé avec une constringence suffisamment faible pour minimiser la puissance de correction chromatique nécessaire, et avec un indice de réfraction suffisamment élevé pour limiter le terme d'aberration sphérique et de coma. La troisième fonction est d'imager la pupille des fibres optiques dans un plan pupillaire le plus proche possible de ces fibres optiques de façon à obtenir une pupille de faible taille, donc un faible diamètre pour la tête optique.

D'une façon générale, les deux blocs optiques de focalisation et de correction participent à la fois à la focalisation et à la correction des aberrations, mais le bloc optique de focalisation est prépondérant dans la focalisation alors que le bloc optique de correction est prépondérant dans la correction des aberrations. Plus précisément, on laisse libre l'aberration dans le bloc optique de focalisation et on élabore un bloc optique de correction dans lequel l'aberration est calculée de façon à corriger l'aberration du bloc optique de focalisation.

Le choix des verres du système optique est fait de manière à rendre le système achromatique sur une large bande spectrale [488nm ;700nm] et ainsi le rendre compatible avec une utilisation en imagerie de fluorescence.

Une lame 1 à faces planes et parallèles en verre BK7, ou K10, a pour fonction de rejeter la réflexion parasite s'opérant en sortie du guide d'image (élément 6 sur la figure 2) en réalisant une adaptation d'indice entre l'indice des coeurs constituant le guide d'image et l'indice du verre BK7, et en déplaçant la réflexion verre-air dans un plan extra-focal. De plus, le choix de la position (plan extra-focal), de la courbure et d'un traitement anti-reflet optimal permet de minimiser les réflexions parasites pouvant venir des autres lentilles. Ceci permet de ne pas parasiter le signal utile provenant de l'échantillon d'analyse dans le cas de l'utilisation de cette tête miniature dans le cadre de l'imagerie de réflectance.

La figure 1 montre schématiquement le trajet optique de faisceaux d'excitation provenant du guide d'image, l'un centré sur l'axe optique du système, les deux autres émergeants de la fibre optique en milieu puis en bord de champ par rapport à l'axe optique du système.

Le faisceau émergeant de la tête converge en un point focal d'excitation situé dans un plan d'analyse subsurfacique. Le signal de fluorescence (ou de réflectance) re-émis par l'échantillon emprunte le même chemin optique en sens inverse avant d'être principalement re-couplé dans la fibre optique d'illumination.

Les caractéristiques détaillées (rayon de courbure, épaisseur, tolérances d'alignement,...) des différentes lentilles ainsi que de la lame de ce premier exemple de réalisation sont données dans le tableau1 ci-dessous :

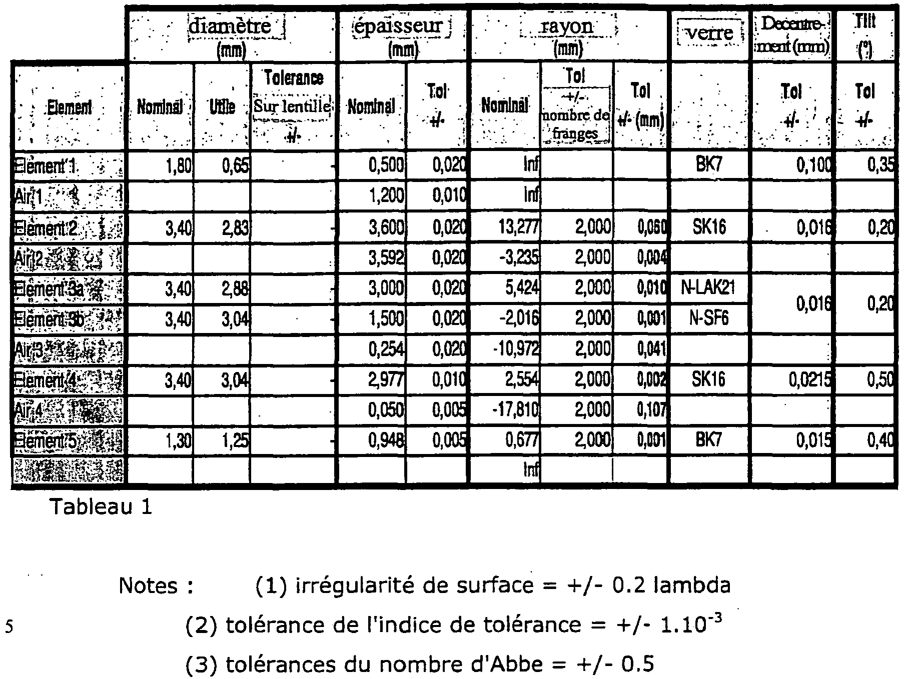

Cet exemple de réalisation permet d'obtenir une très bonne qualité optique et de ce fait une haute résolution spatiale et haute sensibilité. Ses performances sont les suivantes :
- Grandissement = 2,5
- Ouverture numérique image= 0,8 dans l'eau
- Qualité image très proche de la limite de diffraction. L'erreur sur le front d'onde (WFE "Wave Front Error" en anglais) est de λ/15 en centre de champ et λ/10 en bord de champ sur toute la gamme de longueurs d'onde entre 488nm et 700nm. Cette très bonne qualité image assure un bon taux de couplage retour dans la fibre d'excitation (90 %).
- Energie encerclée : elle permet d'évaluer la résolution latérale que l'on est en mesure d'attendre. Dans le cas présent, 50 % de l'énergie provenant du point objet est contenue dans un diamètre de 0,5 µm au centre du champ, et de 1 µm en bord de champ. Dans ce cas, ce n'est pas la tête optique qui limite la résolution latérale, mais la distance entre les coeurs des fibres constituant le guide d'image. La résolution latérale est donnée par la distance inter-coeurs divisée par le grandissement de la tête optique, soit une résolution optique latérale de 1,3 µm.
- Résolution axiale : comprise entre 3 et 5 µm
- Chromatisme axial : 2 µm entre [488nm ;700nm]. Celui-ci est inférieur à la résolution axiale, ce qui engendre donc un minimum de perte de flux de fluorescence.
- Chromatisme latéral : < 0,5 µm entre [488nm ;700nm]. Il est inférieur à la distance entre deux fibres divisée par le grandissement, donc la fibre servant à l'excitation est la même que la fibre de collection.
- Frontale : il s'agit de la distance entre le dernier dioptre optique et le point de focalisation, ce qui correspond à la distance d'observation dans le tissu, puisque la dernière lentille dont la surface est plane est placée en contact avec la surface d'analyse. Dans l'exemple de réalisation, celle-ci vaut 30 µm +/- 10 µm. Différentes valeurs de frontale peuvent être atteintes en modifiant uniquement l'épaisseur de la dernière lentille L5, la tête optique conservant ses performances. A partir de l'exemple de réalisation détaillé dans le tableau 1, on obtient une augmentation de frontale de 10 µm pour une diminution de l'épaisseur géométrique de la lentille L5 de 10 µm environ.
- Champ d'observation : le champ d'observation est défini comme le diamètre utile total du guide d'image divisé par le grandissement du système optique, soit dans ce cas un champ d'observation de diamètre 240 µm.
- Transmission : elle est de l'ordre de 95 % grâce à l'utilisation d'un traitement anti-reflet optimal sur la bande [488nm ;700nm].

Les moyens optiques de la figure 1 sont à intégrer dans un tube porte-optique, formant tête optique, tel qu'illustré sur la figure 2. L'ensemble mécanique représenté sur la figure 2 est constitué par :
- d'un tube métallique de diamètre 4,2mm et de longueur 22,7mm présentant un premier épaulement dans lequel vient s'insérer le guide d'image 6 avec une férule en silice 8 à son extrémité, et un second épaulement dans lequel viennent s'insérer les éléments optiques 2 et 3. La tolérance H6 (-0, +8 µm) sur le diamètre intérieur du tube permet d'assurer un centrage et un tilt de l'élément optique 3 compatible avec les spécifications données dans le tableau1 ci-dessus.
- d'une entretoise mécanique dont la tolérance sur le diamètre extérieur de (-4, -9 µm), la coaxialité et la perpendicularité de 5 µm permettent d'assurer le centrage et le positionnement en tilt de l'élément optique 2.
- d'une pièce mécanique 10 qui permet d'aligner les lentilles 4 et 5 avec le reste de l'ensemble optique. La tolérance H6 (-0, +8 µm) sur le diamètre intérieur et g5 (-4, -9 µm) sur le diamètre extérieur du tube permet d'assurer le centrage de la lentille 4 qui vient en appui au niveau de l'épaulement et est ensuite fixé par collage. Afin de respecter les tolérances d'alignement en tilt de la lentille 5, celui-ci est réglé sur un banc par autocollimation sur sa face plane.
- un support métallique 7 dont le filetage est identique à celui réalisé à l'intérieur du tube côté guide d'image permet d'assembler le guide d'image équipé d'une férule 8 en silice avec la tête opto-mécanique. Toutes ces pièces mécaniques sont réalisées en Inox 316L (matériau biocompatible).

Les dimensions de la tête opto-mécanique présentée dans cet exemple sont de 4,2 mm de diamètre et de 27 mm de longueur de partie rigide, ce qui présente une miniaturisation suffisante pour une manipulation simple et une invasivité minimale, voir nulle dans le cas d'une imagerie par contact avec la surface d'analyse.

Sur la figure 3, on voit un schéma optique d'une tête miniature de grandissement 4.

Cet exemple comparatif de dimensions plus faibles que le premier exemple présente l'avantage de pouvoir être utilisé in vivo par insertion dans le canal opérateur d'un endoscope. De plus, le grandissement de 4 (soit une ouverture numérique de 1,2 dans l'eau) permet d'obtenir :
- une meilleure résolution spatiale (latérale comme axiale) de l'ordre de 1 µm,
- une meilleure sensibilité liée à une ouverture numérique image plus grande (Ouverture numérique de 1,2 dans l'eau).

Cette solution est plus complexe puisqu'elle travaille à plus forte ouverture numérique et dans un encombrement plus réduit ce qui, a priori, est contraire à l'utilisation avec une forte ouverture numérique. L'utilisation d'un grand nombre de lentilles permet d'atteindre les performances requises en amenant progressivement le faisceau dans le bloc de focalisation pour lui donner la bonne ouverture numérique d'éclairement.

La tête optique représentée sur la figure 3 est constituée d'un ensemble optique de neuf lentilles et d'une lame 11 à faces planes et parallèles. Cet ensemble optique est scindé en deux blocs
- le bloc de focalisation est constitué par un doublet 17 achromatique en verre BK7 et SF6, d'une lentille 18 plan-convexe en verre BK7, d'une lentille 19 bi-convexe en BK7 et d'une lentille demi-boule 10 en verre BK7 permettant d'assurer une focalisation importante et un bon contact avec la surface d'analyse.
- le bloc optique de correction permettant de corriger les aberrations induites par le bloc optique de focalisation est constitué d'une lentille 12 biconcave en SF6, d'une lentille 13 bi-convexe en verre BK7, d'une lentille 14 bi-concave en verre SF6, d'une lentille 15 plan-concave en SF6, et d'une lentille 16 plan-concave en BK7. Ce bloc optique correcteur est beaucoup plus complexe puisque la tête présente une très forte ouverture numérique, un même champ d'observation et un encombrement plus réduit.

Le choix des verres du système optique est fait de manière à rendre le système achromatique sur une large bande spectrale [488nm ; 700nm] et ainsi le rendre compatible avec une utilisation en imagerie de fluorescence.

De même que pour l'exemple précédent, la lame à faces planes et parallèles en BK7 a pour fonction de rejeter la réflexion parasite s'opérant en sortie du guide d'image en réalisant une adaptation d'indice entre l'indice des coeurs constituant le guide d'image et l'indice du BK7, et en déplaçant la réflexion verre-air dans un plan extra-focal. De plus, le choix de la position (plan extra-focal), de la courbure et d'un traitement anti-reflet optimal permet de minimiser les réflexions parasites pouvant venir des autres lentilles. Ceci permet de ne pas parasiter le signal utile provenant de l'échantillon d'analyse dans le cas de l'utilisation de cette tête miniature dans le cadre de l'imagerie de réflectance.

La figure 3 montre schématiquement le trajet optique de faisceaux d'excitation provenant du guide d'image, l'un centré sur l'axe optique du système, les deux autres émergeants de la fibre optique en milieu et en bord de champ par rapport à l'axe optique du système.

Le faisceau émergeant de la lame converge en un point focal d'excitation situé dans un plan d'analyse subsurfacique. Le signal de fluorescence (ou de réflectance) réémis par l'échantillon emprunte le même chemin optique en sens inverse avant d'être principalement re-couplé dans la fibre optique d'illumination.

Les caractéristiques détaillées (rayon de courbure, épaisseur,...) des différentes lentilles ainsi que de la lame de ce second exemple de réalisation sont données dans le tableau 2 ci-dessous.

Cet exemple comparatif permet d'obtenir une très bonne qualité optique et de ce fait une haute résolution spatiale et haute sensibilité. Ses performances sont les suivantes :
- Grandissement = 4
- Ouverture numérique image= 1,2 dans l'eau
- Qualité image très proche de la limite de diffraction. L'erreur sur le front d'onde (WFE "Wave Front Error" en anglais) est de λ/30 en centre de champ et λ/15 en bord de champ sur toute la gamme de longueurs d'onde entre 488nm et 700nm. Cette très bonne qualité image assure un taux de couplage retour dans la fibre d'excitation supérieur à 90 %.
- Energie encerclée : dans cet exemple, 50 % de l'énergie provenant du point objet est contenue dans un diamètre de 0.34 µm au centre du champ et de 0.52 µm en bord de champ.
- Résolution axiale : de l'ordre de 2 µm
- Chromatisme axial : 1.2 µm entre [488nm ;700nm]. Celui-ci est inférieur à la résolution axiale, ce qui engendre donc un minimum de perte de flux de fluorescence.
- Chromatisme latéral : 0.1 µm
- Frontale : elle vaut 30 µm +/- 10 µm. De même que la réalisation précédente, cette réalisation met en oeuvre une tête optique avec une frontale ou distance d'observation fixe.
- Champ d'observation : il est dans ce cas de diamètre 150 µm.
- Transmission : compte tenu du nombre de lentilles, elle est de l'ordre de 80 % grâce à l'utilisation d'un traitement anti-reflet optimal sur la bande [488nm ;700nm].
- Diamètre optique : 2 mm
- Longueur optique : 8,6 mm.

Ces dimensions rendent cette solution compatible avec les dimensions de la plupart des canaux opérateurs des endoscopes.

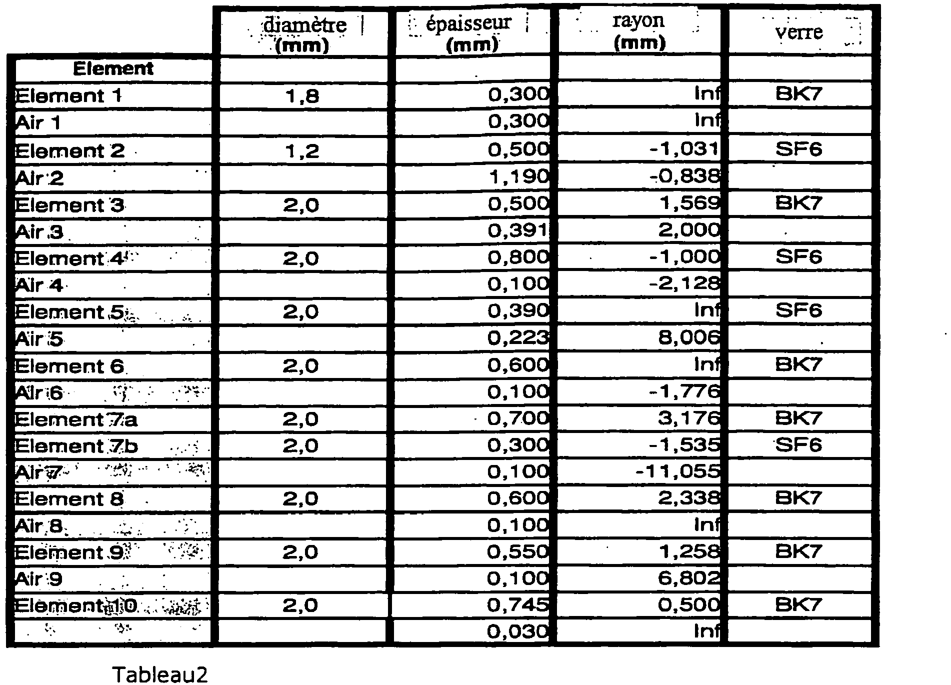

La tête optique selon l'invention procure une correction du chromatisme qui permet d'optimiser la sensibilité suivant deux axes :

### 1. Correction du chromatisme latéral

Cette correction est nécessaire afin que le signal de fluorescence soit couplé au retour dans la fibre optique ayant servie à l'illumination de l'échantillon, et non dans une fibre adjacente qui est filtrée spatialement par le trou de filtrage se trouvant devant le détecteur du dispositif auquel sera connectée la sonde. De ce fait, le chromatisme latéral est inférieur à la distance inter-coeurs divisée par le grandissement de la tête optique.

### 2. Correction du chromatisme axial

Une présence de chromatisme axial dans la tête optique se traduirait directement par une perte de sensibilité par couplage retour dans la fibre optique d'illumination, puisque les photons de fluorescence proviendraient d'une position en Z qui serait différente de la position en Z du faisceau d'illumination, et seront ainsi filtrés par la fibre d'illumination. Afin de s'affranchir de cette perte de sensibilité, le chromatisme axial est inférieur à la résolution axiale.

La tête optique permet également une transmission optimale et une minimisation des réflexions parasites. En effet, la haute sensibilité de la tête optique passe également par l'optimisation de la transmission aller aux longueurs d'onde d'excitation et de la transmission retour aux longueurs d'onde de fluorescence, ce qui est d'autant plus important pour des solutions mettant en oeuvre des solutions optiques complexes constituées de plusieurs lentilles. A cet effet, chaque lentille possède un traitement anti-reflet optimal (R<0,5 %) aux longueurs d'onde de travail. De plus, chaque lentille est placée dans un plan extra-focal et présente une courbure qui permet de rejeter le signal réfléchi à longueur d'excitation en dehors de la fibre d'excitation. Le taux de couplage du signal réfléchi par les lentilles est inférieur à 10⁻⁵ par rapport au signal en sortie de fibre afin d'éviter que le signal provenant de l'échantillon observé ne soit parasité par le signal réfléchi. Ce dernier point est surtout nécessaire dans le cadre de l'imagerie de réflectance.

En ce qui concerne la miniaturisation, les exigences en terme de dimensions sont différentes en fonction du domaine d'application. On distingue deux cas :
1. Imagerie cellulaire ex-vivo ou in vivo sur le petit animal ou sur l'homme nécessitant une imagerie par contact ou à invasivité réduite (laparoscopie,...) Ce besoin nécessite tout de même un dimensionnement minimal (inférieur aux dimensions d'un objectif de microscope) pour des raisons de facilité de manipulation, à savoir un diamètre inférieur à 5 mm et une longueur de la partie rigide de la tête inférieure à 27mm.
2. Imagerie in vivo sur le petit animal ou sur l'homme par voie endoscopique. Ce besoin nécessite des dimensions compatibles avec les dimensions du canal opérateur de l'endoscope dans lequel sera insérée la sonde. Une sonde dont les dimensions sont inférieures à 2,8mm en diamètre et 15mm en longueur de partie rigide est compatible avec la plupart des canaux opérateurs des endoscopes (gastroscope, coloscope, ...).

D'une façon générale, les sondes élaborées selon la présente invention présentent de nombreuses qualités :

### Haute sensibilité :

Ce sont des sondes qui peuvent travailler en profondeur tout en assurant une très bonne collection du signal de fluorescence. En effet, de part leur ouverture numérique côté échantillon très élevée et un ensemble d'optiques corrigeant les aberrations, ces sondes permettent d'exciter l'échantillon avec une importante densité d'énergie et elles permettent également de collecter le maximum de photons de fluorescence en retour.

### Haute résolution spatiale :

Le grandissement optique et la présence d'un ensemble d'optiques corrigeant les aberrations en centre et en bord de champ permettent d'illuminer les tissus avec une PSF ("Point Spread Function") de l'ordre du micron.

Une grande ouverture numérique image combinée à l'utilisation d'optiques corrigeant l'aberration sphérique permet d'illuminer l'échantillon avec un plan de coupe de quelques microns d'épaisseur, ce qui confère aux sondes une très bonne résolution axiale de l'image (capacité de plan de coupe confocale). Ces nouvelles sondes ont donc une bien meilleure résolution axiale, inférieure à 5 µm, contrairement aux sondes Grins par exemple qui atteignent des valeurs de 15 à 20 µm.

### Achromatique :

Les problèmes de chromatisme inhérent au travail en fluorescence sont totalement pris en compte par l'utilisation de verres spécifiques. Les sondes à base de lentilles Grins qui constituent la base technologique de plusieurs acteurs du domaine ne permettent pas de corriger ces effets de chromatisme, ce qui entraîne des pertes de sensibilité puisque le plan d'excitation et le plan d'émission de fluorescence sont spatialement décalés, ce qui se traduit alors par une perte de couplage retour dans la fibre optique.

Miniature Ce sont des sondes qui présentent un design compatible d'une miniaturisation importante, nécessaire pour les applications in vivo et pour notamment être insérée dans le canal opérateur d'un endoscope.

Bien sûr, l'invention n'est pas limitée aux exemples qui viennent d'être décrits et de nombreux aménagements peuvent être apportés à ces exemples sans sortir du cadre de l'invention.

## Revendications

1. Tête optique miniaturisée prévue pour équiper l'extrémité distale d'un faisceau de fibres optiques souples balayé par un faisceau laser, ladite tête optique étant destinée à venir au contact d'un échantillon et exciter ledit échantillon de manière confocale; cette tête optique comprenant un bloc optique de correction des aberrations sphériques et un bloc optique de focalisation;
**caractérisée en ce que** le bloc de focalisation comprend :
- au moins une première lentille (4 ; 18, 19) convergente associée à une lentille (5 ; 10) boule ou demi-boule disposée à l'extrémité distale de la tête optique, et
- des moyens de correction du chromatisme axial et latéral pourvus d'une unique lentille divergente (3b) dont une courbure est centrée sur la pupille du faisceau de fibres optiques; cette lentille divergente étant associée à une seconde lentille convergente (3a) sous forme d'un doublet (3 ; 17)
le bloc de correction des aberrations sphériques comprenant une troisième lentille (2) convergente;
les épaisseurs des première (4) et troisième (2) lentilles convergentes étant agencées pour corriger l'aberration sphérique cumulée sur les dioptres convergents,
l'épaisseur de la troisième lentille (2) convergente étant agencée pour imager la pupille sur la seconde lentille (3) convergente.

2. Tête optique selon la revendication 1, **caractérisée en ce que** le verre de la troisième lentille (2) est déterminé avec une constringence minimisant la puissance de correction chromatique nécessaire, et avec un indice de réfraction limitant le terme d'aberration sphérique et de coma.

3. Tête optique selon l'une quelconque des revendications précédentes, **caractérisée en ce que** ledit doublet (3) comporte un couple de verres d'écart d'indice et d'écart de dispersion chromatique compensant les aberrations chromatiques des lentilles convergentes.

4. Tête optique selon l'une quelconque des revendications précédentes, **caractérisée en ce que** la lentille divergente est courbée de façon à obtenir une ouverture numérique supérieure à 0.8.

5. Tête optique selon l'une quelconque des revendications précédentes, **caractérisée en ce que** les première (4) et troisième (2) lentilles convergentes sont obtenues chacun à partir d'une lentille ayant un petit rayon et un grand rayon, ledit grand rayon étant rendu plan de façon à obtenir une lentille plan-convexe.

6. Tête optique selon l'une quelconque des revendications précédentes, **caractérisée en ce que** la première lentille (4) convergente est conçue et disposée de façon à rejeter l'image de la pupille en avant pour minimiser l'astigmatisme généré par la lentille (5) boule ou demi-boule dans le champ imagé.

7. Tête optique selon l'une quelconque des revendications précédentes, **caractérisée en ce que** la lentille (5) demi-boule est fabriquée dans une bille, dont la face de sortie plane est obtenue par usure sur un polissoir plan.

8. Tête optique selon l'une quelconque des revendications précédentes, **caractérisée en ce que** l'épaisseur de la lentille (5) demi-boule est ajustée pour obtenir une frontale prédéterminée de la tête optique.

9. Tête optique selon la revendication 8, **caractérisée en ce que** la position axiale de la lentille demi boule (5) est déterminée en fonction de l'épaisseur de la dite lentille demi-boule (5) afin d'optimiser les performances optiques de la tête optique pour ladite frontale.

10. Tête optique selon l'une quelconque des revendications précédentes, **caractérisée en ce qu'**elle comprend en outre une lame (1) à faces planes pour rejeter la réflexion parasite s'opérant en sortie du faisceau de fibres optiques.

11. Tête optique selon l'une quelconque des revendications précédentes, **caractérisée en ce que** le signal collecté par la tête optique en provenance de l'échantillon est un signal de fluorescence.

12. Tête optique selon l'une quelconque des revendications 1 à 10, **caractérisée en ce que** le signal collecté par la tête optique en provenance de l'échantillon est un signal de réflectance.

13. Tête optique selon l'une quelconque des revendications précédentes, **caractérisée en ce que** le faisceau de fibres optiques est balayé par le faisceau laser en temps réel de façon à acquérir au moins douze images par seconde.

## Claims

1. Miniaturized optical head provided to equip the distal end of a bundle of flexible optical fibres scanned by a laser beam, said optical head being intended to come into contact with a sample and excite said sample in a confocal manner; this optical head comprising an optical block for correcting spherical aberrations and a focusing optical block; **characterized in that** the focusing block comprises:
- at least a first convergent lens (4; 18, 19) combined with a spherical or hemispherical lens (5; 10) arranged at the distal end of the optical head, and
- means for correcting axial and lateral chromatism that are provided with a single divergent lens (3b) having a curvature centred on the pupil of the bundle of optical fibres; this divergent lens being combined with a second convergent lens (3a) in the form of a doublet (3; 17)
the block for correcting spherical aberrations comprising a third convergent lens (2);
the thicknesses of the first (4) and third (2) convergent lenses being arranged for correcting the cumulative spherical aberration on the convergent dioptres,
the thickness of the third convergent lens (2) being also arranged for imaging the pupil on the second convergent lens (3).

2. Optical head according to claim 1, **characterized in that** the glass of the third lens (2) is determined with a constringence minimizing the necessary chromatic correction power, and with a refractive index limiting the spherical and coma aberration effect.

3. Optical head according to any one of the previous claims, **characterized in that** said doublet (3) comprises a pair of glasses with an index difference and a chromatic dispersion difference compensating for the chromatic aberrations of the convergent lenses.

4. Optical head according to any one of the previous claims, **characterized in that** the divergent lens is curved so as to obtain a numerical aperture greater than 0.8.

5. Optical head according to any one of the previous claims, **characterized in that** the first (4) and third (2) convergent lenses are each obtained from a lens having a small radius and a large radius, said large radius being made plane so as to obtain a plano-convex lens.

6. Optical head according to any one of the previous claims, **characterized in that** the first convergent lens (4) is designed and arranged so as to reject the image of the pupil ahead to minimize the astigmatism generated by the spherical or hemispherical lens (5) in the imaged field.

7. Optical head according to any one of the previous claims, **characterized in that** the hemispherical lens (5) is produced in a ball, the plane output face of which is obtained by abrasion on a plane polisher.

8. Optical head according to any one of the previous claims, **characterized in that** the thickness of the hemispherical lens (5) is adjusted in order to obtain a predetermined frontal area of the optical head.

9. Optical head according to claim 8, **characterized in that** the axial position of the hemispherical lens (5) is determined according to the thickness of said hemispherical lens (5) in order to optimize the optical performances of the optical head for said frontal area.

10. Optical head according to any one of the previous claims, **characterized in that** it also comprises a plate (1) with plane faces for eliminating the stray reflection occurring at the output of the bundle of optical fibres.

11. Optical head according to any one of the previous claims, **characterized in that** the signal collected by the optical head that originates from the sample is a fluorescence signal.

12. Optical head according to any one of claims 1 to 10, **characterized in that** the signal collected by the optical head that originates from the sample is a reflectance signal.

13. Optical head according to any one of the previous claims, **characterized in that** the bundle of optical fibres is scanned by the laser beam in real time so as to acquire at least twelve images per second.

## Patentansprüche

1. Miniaturisierter optischer Kopf, der zum Bestücken des distalen Endes eines Bündels von flexiblen optischen Fasern vorgesehen ist, das von einem Laserstrahl abgetastet wird, wobei der optische Kopf dazu bestimmt ist, mit einer Probe in Kontakt zu gelangen und die Probe konfokal anzuregen, wobei dieser optische Kopf einen optischen Korrekturblock zur Korrektur von sphärischen Aberrationen und einen optischen Fokussierungsblock aufweist, **dadurch gekennzeichnet, dass** der Fokussierungsblock aufweist:
- zumindest eine erste Sammellinse (4; 18, 19), der eine kugelförmige bzw. halbkugelförmige Linse (5; 10) zugeordnet ist, die am distalen Ende des optischen Kopfes angeordnet ist, und
- Korrekturmittel zur Korrektur der axialen und lateralen chromatischen Aberration, die mit einer einzelnen Streulinse (3b) ausgestattet sind, von welcher eine Krümmung auf die Pupille des Bündels von optischen Fasern zentriert ist, wobei dieser Streulinse eine zweite Sammellinse (3a) in Form eines Dublets (3; 17) zugeordnet ist,
wobei der Korrekturblock zur Korrektur von sphärischen Aberrationen eine dritte Sammellinse (2) aufweist;
wobei die Dicken der ersten (4) und dritten (2) Sammellinse dazu eingerichtet sind, die kumulative sphärische Aberration an den bündelnden Linsenflächen zu korrigieren,
wobei die Dicke der dritten konvergierenden Linse (2) dazu eingerichtet ist, die Pupille auf der zweiten konvergierenden Linse (3) abzubilden.

2. Optischer Kopf nach Anspruch 1, **dadurch gekennzeichnet, dass** das Glas der dritten Linse (2) mit einer Abbeschen Zahl, welche die erforderliche Leistung der chromatischen Korrektur minimiert, und mit einem Brechungsindex bestimmt wird, der die sphärische Aberration und die Koma begrenzt.

3. Optischer Kopf nach einem der vorangehenden Ansprüche, **dadurch gekennzeichnet, dass** das Dublet (3) ein Paar von Gläsern mit unterschiedlichem Brechungsindex und unterschiedlicher chromatischer Dispersion aufweist, wodurch die chromatischen Aberrationen der konvergierenden Linsen ausgeglichen werden.

4. Optischer Kopf nach einem der vorangehenden Ansprüche, **dadurch gekennzeichnet, dass** die Streulinse so gekrümmt ist, dass eine numerische Apertur von über 0,8 erhalten wird.

5. Optischer Kopf nach einem der vorangehenden Ansprüche, **dadurch gekennzeichnet, dass** die erste (4) und die dritte (2) Sammellinse jeweils ausgehend von einer Linse mit einem kleineren Radius und einem größeren Radius erhalten werden, wobei der größere Radius abgeflacht wird, so dass eine plankonvexe Linse erhalten wird.

6. Optischer Kopf nach einem der vorangehenden Ansprüche, **dadurch gekennzeichnet, dass** die erste Sammellinse (4) so ausgelegt und angeordnet ist, dass sie das Bild der Pupille nach vorne wirft, um den Astigmatismus zu minimisieren, der von der kugelförmigen bzw. halbkugelförmigen Linse (5) in dem Bildfeld erzeugt wird.

7. Optischer Kopf nach einem der vorangehenden Ansprüche, **dadurch gekennzeichnet, dass** die halbkugelförmige Linse (5) aus einer Kugel hergestellt ist, deren ebene Austrittsfläche durch Abrieb an einem ebenen Polierwerkzeug erhalten wird.

8. Optischer Kopf nach einem der vorangehenden Ansprüche, **dadurch gekennzeichnet, dass** die Dicke der halbkugelförmigen Linse (5) so abgestimmt ist, dass eine vorbestimmte Frontlinse des optischen Kopfes erhalten wird.

9. Optischer Kopf nach Anspruch 8, **dadurch gekennzeichnet, dass** die axiale Stellung der halbkugelförmigen Linse (5) in Abhängigkeit von der Dicke der halbkugelförmigen Linse (5) bestimmt wird, um die optische Leistung des optischen Kopfes für die Frontlinse zu optimieren.

10. Optischer Kopf nach einem der vorangehenden Ansprüche, **dadurch gekennzeichnet, dass** er ferner eine Zunge (1) mit ebenen Flächen aufweist, um die Störreflexion zurückzuwerfen, die am Austritt des Bündels von optischen Fasern erfolgt.

11. Optischer Kopf nach einem der vorangehenden Ansprüche, **dadurch gekennzeichnet, dass** das von der Probe stammende Signal, das von dem optischen Kopf erfasst wird, ein Fluoreszenzsignal ist.

12. Optischer Kopf nach einem der Ansprüche 1 bis 10, **dadurch gekennzeichnet, dass** das von der Probe stammende Signal, das von dem optischen Kopf erfasst wird, ein Reflexionssignal ist.

13. Optischer Kopf nach einem der vorangehenden Ansprüche, **dadurch gekennzeichnet, dass** das Bündel optischer Fasern von dem Laserstrahl in Echtzeit so abgetastet wird, dass mindestens zwölf Bilder pro Sekunde erhalten werden.
